# FASCICULE DE BREVET EUROPEEN

(11) **EP 1 438 278 B1**
(45) Date de publication et mention de la délivrance du brevet: **27.02.2013**
(21) Numéro de dépôt: 02790512.4
(22) Date de dépôt: 15.10.2002
(51) Int. Cl.: C07C 17/25, C07C 17/38, C07C 21/06, B01D 1/22, B01D 3/14

(54) **PROCEDE D'OBTENTION DE CHLORURE DE VINYLE POLYMERISABLE A PARTIR D'UN PRODUIT BRUT ISSU DE LA PYROLYSE DU 1,2-DICHLOROETHANE**
VERFAHREN ZUR GEWINNUNG EINES POLYMERISIERBAREN VINYLCHLORIDS AUS DEM ROHPRODUKT EINER 1,2-DICHLORETHAN-PYROLYSE
METHOD FOR OBTAINING POLYMERISABLE VINYL CHLORIDE FROM A RAW PRODUCT DERIVED FROM PYROLYSIS OF 1,2-DICHLOROETHANE

(30) Priorité: 17.10.2001 FR 0113364
(43) Date de publication de la demande: 21.07.2004
(73) Titulaire: Kem One, 69007 Lyon (FR)
(72) Inventeur: RONDOT, Béatrice, F-30640 Beauvoisin (FR); VANNEY, François, F-69006 Lyon (FR)
(74) Mandataire: Colombet, Alain André
(86) Numéro de dépôt international: PCT/FR2002/003518
(87) Numéro de publication internationale: WO 2003/033443

(56) Documents cités:
- EP-A- 0 073 941
- GB-A- 1 218 224

## Description

L'invention concerne un procédé d'obtention de chlorure de vinyle apte à la polymérisation, à partir d'un produit brut issu de la pyrolyse du 1,2-dichloroéthane.

L'invention concerne aussi un dispositif pour la mise en oeuvre du procédé.

Les produits de la pyrolyse du 1,2-dichloroéthane ci-après désigné par DCE contiennent notamment de faibles quantités de 1,3-butadiène, de l'ordre de 10 à 100 parties par million (ppm), rapportées au chlorure de vinyle. Ces teneurs en 1,3-butadiène désigné ci-après par BD qui se retrouvent dans le chlorure de vinyle final sont suffisantes pour inhiber sa polymérisation et il est nécessaire de diminuer les teneurs en BD à moins de 8 ppm pour rendre le chlorure de vinyle polymérisable dans des conditions industrielles.

On a souvent constaté que la teneur en BD formé lors de la pyrolyse du DCE était d'autant plus faible que ledit DCE mis en oeuvre était plus pur, mais il est toujours nécessaire de limiter la teneur en BD dans le chlorure de vinyle obtenu par pyrolyse du DCE pour que la polymérisation du chlorure de vinyle puisse se dérouler normalement.

Pour ce faire, de nombreux procédés ont été proposés. Dans la majorité des cas, ces procédés mettent en jeu, pour éliminer le BD, des réactions de chloration, d'hydrochloration ou bien d'hydrogénation en présence ou non de catalyseurs.

Ainsi, le brevet FR 1216030 propose d'épurer le chlorure de vinyle obtenu par pyrolyse du DCE, en faisant passer le chlorure de vinyle en mélange avec du chlorure d'hydrogène, en phase vapeur, sur un catalyseur d'hydrochloration tel que HgCl₂ ou FeCl₃. Ce procédé exige cependant d'inclure une installation de traitement catalytique supplémentaire, ce qui peut accroître sensiblement les frais de production, et exige qu'au moins une partie du DCE non converti soit séparée des produits de la pyrolyse.

Dans le brevet US 3125607, on ajoute du chlore anhydre au chlorure de vinyle à une température comprise entre -20°C et 0°C selon un rapport chlore sur BD à éliminer d'environ 5/1. La teneur en BD passe de 200 ppm à quasiment 0 ppm après 30 minutes de chloration.

Ce procédé présente cependant l'inconvénient d'inclure une distillation supplémentaire pour éliminer les produits chlorés du BD, le chlore en excès et diverses impuretés susceptibles d'être formées lors de la chloration du BD.

Le brevet US 3125608 décrit un procédé de purification du chlorure de vinyle gazeux, contenant du BD en chauffant ledit chlorure de vinyle avec de l'hydrogène à une température allant de 60°C à 250°C en présence d'un catalyseur constitué de Pd supporté sur alumine activée.

Ce procédé présente l'inconvénient d'inclure une installation d'hydrogénation supplémentaire.

Selon le procédé décrit dans le brevet US 3142709, on épure en BD le chlorure de vinyle en mettant le chlorure de vinyle liquide au contact d'une quantité de chlorure d'hydrogène anhydre, représentant de 0,5 à 5 % en poids du chlorure de vinyle, pendant une période allant de 2 minutes à 5 heures. Ce procédé ne s'applique toutefois, qu'au chlorure de vinyle déjà séparé des autres produits de la pyrolyse et d'effectuer une distillation supplémentaire.

Dans le brevet FR 1602522, on décrit un procédé d'obtention de chlorure de vinyle directement apte à la polymérisation, qui consiste à partir du produit brut issu de la pyrolyse du DCE, à condenser sous une pression supérieure à la pression atmosphérique, ledit produit brut pour obtenir une phase liquide qu'on laisse mûrir deux heures au moins à une température comprise entre 0°C et 100°C, avant d'en séparer les constituants.

D'après ce procédé, la phase liquide après un tel traitement ne contiendrait pratiquement plus de BD. Pratiquement, cette phrase liquide séjourne dans des réservoirs.

Ce procédé s'applique tout particulièrement à des produits de pyrolyse contenant au plus 10 ppm de BD par rapport au chlorure de vinyle.

Lorsque les produits de pyrolyse contiennent des quantités de BD plus importantes telles que par exemple au plus 20 ppm, on opère de la façon suivante.

Après l'étape de condensation préalable qui s'effectue par un refroidissement approprié des produits gazeux de pyrolyse, maintenus sous une pression supérieure à la pression atmosphérique, on obtient une phase gazeuse qui est en équilibre avec la phase liquide, et qui contient une certaine quantité de BD. La phase liquide mûrie est utilisée comme liquide de lavage de ladite phase gazeuse, de façon à absorber le BD résiduaire. Elle subit ensuite un nouveau mûrissage de deux heures au moins. Ainsi, en renouvelant cette opération, dans une ou plusieurs colonnes, selon la teneur en BD présent dans le produit brut de pyrolyse, il est possible d'épurer totalement en BD le chlorure de vinyle.

Bien que donnant des résultats satisfaisants, ces différentes façons d'opérer présentent de nombreux inconvénients.

Pour de faibles teneurs en BD, ce procédé ne s'applique qu'à la phase liquide obtenue après la condensation du produit brut issu de la pyrolyse du DCE et ne tient pas compte des teneurs en BD de la phase gazeuse qui peuvent être fluctuantes et par conséquent se retrouver dans le chlorure de vinyle final.

Lorsque les quantités de BD sont plus élevées, on effectue au moins un lavage de là phase gazeuse avec la phase liquide mûrie au moins 2 heures, laquelle phase liquide de lavage est à nouveau mûrie au moins deux heures.

Cette façon de procéder entraîne de nombreux équipements supplémentaires et les temps de séjour longs conduisent à des capacités importantes de produits inflammables et toxiques sous pression.

La demanderesse a trouvé que l'on pouvait obtenir un chlorure de vinyle apte à la polymérisation en s'affranchissant des inconvénients des procédés précédemment mentionnés, en utilisant des moyens simples et efficaces pour réduire le 1,3-butadiène (BD) de la totalité du flux sortant de la pyrolyse du DCE.

L'invention a donc pour objet un procédé d'obtention de chlorure de vinyle directement apte à la polymérisation à partir de la totalité du produit brut issu de la pyrolyse du DCE ayant subi un refroidissement, ledit procédé étant caractérisé en ce que la totalité du produit brut refroidi, constitué d'une phase liquide et d'une phase gazeuse est refroidi à nouveau à une température au plus égale à 40°C, sous une pression allant de 10 à 15 bars puis ensuite récupéré dans une enceinte à des températures et pressions sensiblement identiques dans laquelle la phase liquide séjourne pendant une durée au plus égale à 20 minutes et, de préférence, pendant une durée allant de 5 à 15 minutes, avant la séparation des constituants.

Selon la présente invention, on désigne par chlorure de vinyle apte à la polymérisation un chlorure de vinyle ayant une teneur pondérale en BD au plus égale à 8 ppm et, de préférence, comprise entre 2 et 7 ppm.

Selon la présente invention, le flux gazeux sortant du (des) four(s) de pyrolyse subit un refroidissent approprié qui consiste en une succession de trempes et de condensations qui conduit à un mélange ou la phase gazeuse est en équilibre avec la phase liquide. Ce mélange, produit brut refroidi, comprend du chlorure de vinyle, du chlorure d'hydrogène, du DCE non transformé, du BD et divers sous-produits. Les quantités de BD peuvent varier dans une large mesure. Elles dépendent notamment de la pureté du DCE mis en oeuvre et des conditions de pyrolyse dudit DCE. Elle sont généralement comprises dans les procédés actuels entre 10 ppm et 100 ppm.

Selon la présente invention, la totalité du produit brut refroidi est soumis à nouveau à un refroidissement sous une pression supérieure à la pression atmosphérique dans un échangeur de chaleur type échangeur à film tombant duquel il sort à une température au plus égale à 40°C. La pression régnant à l'intérieur dudit échangeur est au plus égale à 15 bars et, de préférence comprise entre 10 et 15 bars.

La totalité du produit brut refroidi ainsi traité est directement récupéré dans une enceinte sous des conditions de pression et de températures quasiment identiques à celles auxquelles il a été refroidi dans l'échangeur à film tombant, dans laquelle la phase liquide séjourne pendant une durée au plus égale à 20 minutes et, de préférence, pendant une durée allant de 5 à 15 minutes.

Ensuite on effectue la séparation des constituants du mélange selon des moyens connus en soi. Selon la présente invention, on préfère cependant séparer dans une première colonne de distillation le chlorure d'hydrogène du mélange DCE / chlorure de vinyle puis dans une seconde colonne le chlorure de vinyle du DCE et enfin, on élimine les dernières traces d'HCl dans un dispositif approprié.

Le chlorure de vinyle sortant de la troisième colonne présente avantageusement une teneur en BD inférieure à 8 ppm et est alors directement apte à la polymérisation.

L'invention concerne également un dispositif pour la mise en oeuvre du procédé.

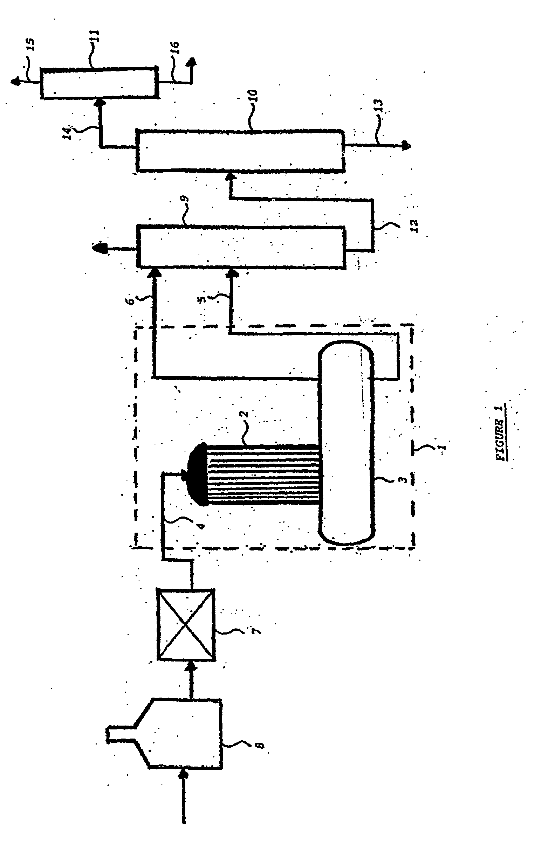

Ce dispositif (1) disposé dans une installation de production de chlorure de vinyle tel que représenté schématiquement sur la figure 1 (zone en pointillé), intercalé entre une zone de refroidissement (7) du flux gazeux (FG) sortant de la zone de pyrolyse (8) et la colonne de distillation HCl (9), est constitué d'un échangeur de chaleur à film tombant (2) surmontant un pot récepteur (3) qui est un réservoir cylindrique horizontal. L'échangeur (2) est alimenté par la totalité du produit brut refroidi (en 7) par une conduite (4). La colonne de distillation HCl est alimentée en produit brut traité dans le dispositif (1) d'une part, en produit liquide par la conduite (5) et, d'autre part en produit gazeux par la conduite (6). De la colonne (9), sort en tête HCl et en pied un mélange, comprenant du chlorure de vinyle, du DCE non transformé et divers sous-produits qui alimente par une conduite (12) la colonne de distillation du chlorure de vinyle (10) de laquelle sort en tête le chlorure de vinyle (14) qui est purifié dans un dispositif approprié (11) de laquelle il sort en (16) et est apte à être polymérisé industriellement.

En (13), sort le DCE non transformé et divers sous produits.

En (15), sort l'HCl résiduel.

Le dispositif selon l'invention est conçu de telle façon que l'échangeur se trouve disposé sur le pot récepteur de façon que les produits de l'échangeur s'écoulent directement dans le dit pot.

L'échangeur à film tombant est constitué de tubes verticaux enfermés dans une calandre dans lesquels circulent les flux liquide et gazeux, le liquide ruisselant le long des parois desdits tubes. Le refroidissement est assuré par de l'eau de refroidissement circulant côté calandre. L'homme du métier déterminera le nombre de tubes nécessaires adaptés aux flux à traiter et au refroidissement.

Le procédé et le dispositif pour le mettre en oeuvre le procédé selon l'invention permettent d'abaisser la teneur en BD dans le chlorure de vinyle, le rendant directement polymérisable. Ils présentent également l'avantage de soulage la demande en frigories en tête de colonne de distillation de HCl. L'exemple qui suit illustre l'invention.

### EXEMPLE

L'exemple est réalisé sur une installation industrielle telle que schématisée sur la figure 1 et dimensionnée d'une façon telle qu'elle puisse produire 50 tonnes par heure de chlorure de vinyle directement polymérisable.

La zone de pyrolyse (8), constituée de 3 fours, est alimentée avec du DCE d'une pureté égale à 99,7 %.

Cette zone est maintenue à une température moyenne d'environ 480°C sous une pression de 30 bars.

Le flux issu de cette zone de pyrolyse, qui comprend du chlorure de vinyle (CV), de l'HCl, du DCE non converti (environ 50 %), du BD et autres sous-produits, sort à une température voisine de 480°C et sous une pression de 30 bars, passe dans une zone de refroidissement (7) constituée de colonnes de trempe qui abaisse sa température vers 180°C et divers échangeurs à divers niveau de froid qui abaissent sa température de 180°C à environ 60°C.

La totalité du produit brut ainsi refroidi qui sort de (7), constitué d'une phase gazeuse comprenant du chlorure de vinyle, HCl et de faibles quantités de DCE et d'une phase liquide, comprenant du DCE, du chlorure de vinyle et de faibles quantités de HCl, est introduit en tête de l'échangeur à film tombant (2) à une température d'environ 56°C. Cet échangeur est constitué de 1652 tubes et est refroidi avec de l'eau à 28°C circulant dans la calandre à contre courant. La pression à l'intérieur de l'échangeur à film tombant (2) est de 14 bars.

Le flux sort de (2) à une température de 37°C sous une pression de 14 bars et tombe directement dans le pot (3) qui est un simple réservoir cylindrique horizontal, dans lequel la phase liquide séjourne 15 minutes à environ 37°C sous une pression de 14 bars. Les flux gazeux et liquide sont ensuite dirigés vers la colonne de distillation (9) respectivement par les conduites (6) et (5). Dans cette colonne, on sépare en tête HCl à environ 25°C sous une pression de 13 bars.

Dans le bouilleur maintenu à environ 110°C on prélève la phase liquide comprenant du CV, du DCE et divers sous-produits qui est dirigée vers la colonne de distillation (10) par la conduite (12).

Dans cette colonne on sépare entête le CV à 30°C sous une pression de 4 bars.

Le pied de la colonne (10) maintenu à 150°C contient essentiellement du DCE et divers sous-produits qui sortent en (13). Le CV est ensuite envoyé dans la colonne (11) par la conduite (14) où l'on élimine l'HCl résiduaire qui sort en tête (15). Le CV sortant de cette colonne en (16) a une teneur moyenne pondérale en BD allant de 3 à 6 ppm et est par conséquent directement polymérisable industriellement.

## Revendications

1. Procédé d'obtention de chlorure de vinyle à partir de la totalité du produit brut issu de la pyrolyse du 1,2-dichloroéthane ayant subi un refroidissement, ledit procédé étant **caractérisé en ce que** le produit brut refroidi, constitué d'une phase liquide et d'une phase gazeuse est refroidi à nouveau à une température au plus égale a 40 C, sous une pression allant de 10 à 15 bars puis ensuite récupéré dans une enceinte à des températures et pressions sensiblement identiques, dans laquelle la phase liquide séjourne pendant une durée au plus égale à 20 minutes, avant la séparation des constituants.

2. Procédé selon la revendication 1, **caractérisé en ce que** la phase liquide séjourne dans l'enceinte pendant une durée allant de 5 à 15 minutes.

3. Procédé selon l'une des revendications 1 ou 2, **caractérisé en ce que** les constituants sont séparés par distillation sous des pressions supérieures à la pression atmosphérique.

4. Dispositif pour la mise en oeuvre du procédé selon l'une des revendications 1 à 3 ; **caractérisé en ce qu'**il est constitué d'un échangeur à film tombant (2) surmontant un pot récepteur (3), ces appareillages étant intercalés entre une zone de refroidissement (7) du flux gazeux sortant de la zone de pyrolyse (8) et une zone de distillation comprenant une colonne de distillation HCl (9), une colonne de distillation du chlorure de vinyle (10), et un dispositif approprié d'élimination de traces d'HCl (11).

## Claims

1. A method for obtaining vinyl chloride from all of the raw product derived from the pyrolysis of 1,2-dichloroethane having undergone a cooling operation, said method being **characterized in that** the cooled raw product, consisting of a liquid phase and a gas phase, is cooled further down to a temperature of at most 40°C under a pressure ranging from 10 to 15 bar, and then recovered in a tank at substantially identical temperatures and pressures, in which tank the liquid phase remains for a time of at most 20 minutes before separation of the constituents.

2. The method as claimed in claim 1, **characterized in that** the liquid phase remains in the tank for a time ranging from 5 to 15 minutes.

3. The method as claimed in either of claims 1 and 2, **characterized in that** the constituents are separated by distillation under pressures greater than atmospheric pressure.

4. An apparatus for implementing the method as claimed in one of claims 1 to 3, **characterized in that** it consists of a falling-film exchanger (2) placed on top of a receiving pot (3), these items of equipment being inserted between a cooling zone (7), for cooling the gas stream coming from the pyrolysis zone (8), and a distillation zone comprising an HCl distillation column (9), a vinyl chloride distillation column (10) and a suitable device (11) for eliminating traces of HCl.

## Patentansprüche

1. Verfahren zur Gewinnung eines Vinylchlorids aus der Gesamtheit des Rohstoffs, der bei der Pyrolyse des 1,2-Dichlorethans entsteht und der eine Kühlung erfahren hat, wobei das Verfahren **dadurch gekennzeichnet ist, dass** das gekühlte Rohprodukt, das aus einer Flüssigphase und einer Gasphase besteht, bei einer Temperatur von höchsten 40°C und einem Druck zwischen 10 und 15 bar erneut gekühlt wird und dann in einem Gehäuse bei im Wesentlichen gleichen Temperaturen und
Drücken zurückgewonnen wird, in dem die Flüssigphase während einer Dauer von höchstens 20 Minuten vor der Trennung der Bestandteile verbleibt.

2. Verfahren nach Anspruch 1, **dadurch gekennzeichnet, dass** die Flüssigphase in dem Gehäuse während einer Dauer zwischen 5 und 15 Minuten verbleibt.

3. Verfahren nach einem der Ansprüche 1 oder 2, **dadurch gekennzeichnet, dass** die Bestandteile durch Destillation unter Drücken größer als dem atmosphärischen Druck getrennt werden.

4. Vorrichtung zur Durchführung des Verfahrens nach einem der Ansprüche 1 bis 3, **dadurch gekennzeichnet, dass** sie aus einem Fallfilmverdampfer (2), der über einem Sammelbehälter (3) montiert ist, gebildet wird, wobei diese Geräte zwischen einer Zone (7) der Kühlung des Gasstroms, der aus der Pyrolysezone (8) austritt, und einer Destillationszone, die eine HCl-Destillationskolonne (9), eine Chlorvinyl-Destillationskolonne (10) und eine geeignete Vorrichtung (11) zur Eliminierung von HCl-Spuren aufweist, eingefügt sind.
